# EUROPEAN PATENT SPECIFICATION

(11) **EP 3 192 818 B1**
(45) Date of publication and mention of the grant of the patent: **28.10.2020**
(21) Application number: 15382625.0
(22) Date of filing: 15.12.2015
(51) Int. Cl.: A61K 8/81, C08F 220/58, C08F 226/10, A61Q 13/00

(54) **A CONTROLLED RELEASE CROSS-LINKED MOULD SHAPED SCENTED PRODUCT**
VERNETZTES, IN EINER FORM GEFORMTES, PARFÜMIERTES PRODUKT MIT KONTROLLIERTER FREISETZUNG
PRODUIT PARFUMÉ RÉTICULÉ FORMÉ DANS UN MOULE À LIBÉRATION CONTRÔLÉE

(43) Date of publication of application: 19.07.2017
(73) Proprietor: Antonio Puig, S.A., 08902 Hospitalet de Llobregat (ES)
(72) Inventor: PANYELLA COSTA, David, 08030 BARCELONA (ES); VIDAL TASA, Jordi, 08030 BARCELONA (ES); MONTANYÀ DE JUAN, Sonia, 08030 BARCELONA (ES)
(74) Representative: ZBM Patents - Zea, Barlocci & Markvardsen

(56) References cited:
- WO-A1-99/04830
- WO-A1-2014/071354
- FR-A1- 2 803 746
- US-A- 3 881 026
- US-A- 4 036 788
- US-A1- 2006 183 822
- US-B2- 6 900 165

## Description

The present invention relates to the field of perfumery and more particularly to the field of cosmetic products and air fresheners. Especially, it relates to a controlled release cross-linked mould shaped scented product containing a cross-linked mould shaped polymer having a fragrance absorbed. The invention also relates to scented articles containing the cross-linked mould shaped scented product, as well as a process for their preparation.

### BACKGROUND ART

The origins of the fragrance industry lie in prehistory. At some point our ancestors, found there was pleasure in the aroma of a flower, and that mixing certain herbs with food added relish. When this took place will never be known, but it could be said that at that point we became truly human. A crucial point in the fragrance industry was that the use of flavorings and fragrances, in whatever form, are a feature of a society. The classical world of ancient Greece and Rome was familiar with many oils, spices, and perfumes. Further, some of the key techniques common to the flavor and fragrance industry had been developed in that time, in particular, distillation and the concept of extraction. In the classical world, a basic concept such as distillation was used to refine a crude material into a more valuable one.

From the early nineteenth century, developments in chemistry began to create the flavor and fragrance industry, as we know it. The purification of natural materials, especially essential oils, led to the identification of aroma-active materials. Nowadays, there is a particular effort in the fragrance industry for the development of novel fragrances and the control of their release (cf. David J. Rowe. "Chemistry and technology of flavors and fragrances". Chapter 1. "Introduction", Blackwell Publishing, 2005, pp.1-11)

Polymer gels (hydrogel) of cross-linked macromolecules capable of forming a three dimensional network have been widely disclosed. These polymer gels absorb water molecules inside the network by osmotic forces. Therefore, they have been used in personal care products. These superabsorbent polymers are capable of absorbing large quantities of water

Therefore, gels in form of soft solids which can entrap organic solvents into the three dimensional matrix have been developed. Examples of gels made of such superabsorbents of organic solvents are chlorosulfonated polyethylene gels (cf. Varma, A. J., et al. "Separations based on chemically selective polymer gels". Chemical Engineering Science, 1995, vol. 50 (23), pp 3835-3838), and polyethylene oxide gels (cf. Graham, N. B., et al., "Interaction of poly(ethylene oxide) with solvents; 1. Preparation and swelling of a crosslinked poly(ethylene oxide) hydrogel".Polymer, 1982, vol. 23, pp. 1345). These soft gels can absorb large quantities of organic solvents transforming the liquid solvent into a gelled solid. The properties of these soft solids are intermediate between solids and liquids becoming them useful for the preparation of pastes, lotions, creams, and gelled fuels.

Particularly, the United States patent number US 6794467 discloses a process for the preparation of a cross-linked polymer powdered absorbent useful for gelling organic liquids. The process comprises mixing one or more monomers with a cross-linking agent with a free radical initiator and subjecting the mixture so obtained to a polymerization method, followed by a crushing step to obtain a polymer in powder form. Then, the polymer in form of powder is swelled with alcohols to yield gellified polymeric powder. Those gellified polymeric powders have been used for the preparation of gelled fuels. These fuels are particularly useful because they are easier and safer to transport, and allows a slower diffusive release of the fuel from the gelled matrix.

In the field of perfumery, soft-gelled perfumes forming a three dimensional matrix wherein the perfume is entrapped have been disclosed. Particularly, the European patent application number EP181401 discloses a process for their preparation which comprises adding a perfume oil to an alcohol solution containing hydroxypropylcellulose with a saturated solution of dibenzylidenesorbitol in N-methyl-2-pyrrolidine. Similarly FR2803746A1 discloses a process for the preparation of an air freshener composition which comprises a perfume, a solubilizing agent, an aqueous medium and a superabsorbent polymer being a crosslinked acrylate or acrylamide/acrylate copolymer.

Therefore, from what is known in the art, it is derived that there is still the need of providing a prolonged release polymeric product capable of super-absorbing alcoholic fragrances inside their matrix for long lasting scents.

### SUMMARY OF THE INVENTION

Inventors have found that the cross-linked mould shaped polymer of the invention is capable of super-absorbing alcoholic fragrances, and allowing the controlled/prolonged release of those fragrances. As it is illustrated in the examples, the absorption of the fragrance inside the cross-linked mould shaped polymer of the invention allows prolonging the release of the fragrance providing a continuous pleasant organoleptic effect. The cross-linked mould shaped scented product of the invention is also advantageous because it can be incorporated in scented articles.

Thus, the first aspect of the present invention relates to a cross-linked mould shaped scented product as defined in claim 1.

The second aspect of the present invention relates to a process for the preparation of the cross-linked mould shaped scented product as defined in the first aspect of the invention.

The third aspect of the invention relates to a scented article comprising the cross-linked mould shaped scented product as defined in the first aspect of the invention.

The fourth aspect of the invention relates to the use of the cross-linked mould shaped scented product of the first aspect of the invention; or alternatively the scented article as defined in the third aspect of the invention for perfuming.

### DETAILED DESCRIPTION OF THE INVENTION

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

The term "cross-linked mould shaped polymer" refers to a tri-dimensional (3D) compound containing the polymer, which is in solid form. In particular, these compounds, maintain its shape when an alcoholic fragrance is absorbed giving rise to a cross-linked shaped scented product.

The term "cross-linked mould shaped scented product" refers to a tri-dimensional (3D) product containing the cross-linked mould shaped polymer.

The term "alcoholic fragrance" refers to a fragrance that includes one or more alcohols in combination with one or more essential oils

The term "perfume" as used herein refers to eau de perfumes, eau de colognes, eau de toilettes, and other compositions containing fragrances.

The term "percentage (%) by weight of the weight of the product" refers to the percentage of each ingredient in relation to the total weight of the product.

The term "mol (%)" as used herein refers to the mole fraction or molar fraction, which is the amount of an ingredient expressed in moles, divided by the total amount of monomer (a1) and monomer (a2) mols of all ingredients of the mixture.

The term "appropriate acceptable" refers to that excipients or carriers suitable for use in perfumery, particularly in the cosmetic and freshener field for the preparation of scented articles with perfumery use.

The term "scented article" refers to any product that is intended for consumers and contains the cross-linked mould shaped scented product.

The cross-linked mould shaped polymer (a) is obtainable by a process as defined above in the first aspect of the invention. Thus, its preparation process may define the cross-linked mould shaped polymer (a). For the purposes of the invention the expressions "obtainable", "obtained" and equivalent expressions are used interchangeably, and in any case, the expression "obtainable" encompasses the expression "obtained".

Polymerisation is conducted inside a mould in the presence of the cross-linker, a polymerization initiator, and a polymerization catalyst. In an embodiment, the polymerisation can be carried out by thermal polymerisation, and in an alternative embodiment, the polymerisation can be carried out by photochemical polymerisation.

Generally, when the process is carried out by thermal polymerisation inside the mould, the temperature is comprised from 50°C to 90°C; preferably comprised from 60°C to 80°C.

The polymerisation process of the invention is carried out in the presence of a polymerization initiator. The term "polymerization initiator" refers to a substance that reacts with the vinyl monomers to form an intermediate, thereby inducing polymerization initiation. When the polymerization is a thermal polymerisation, then the polymerization initiator is activated by supplying thermal energy. Examples of thermal polymerization initiator suitable for the present invention include, but not limited to, ammonium persulfate, potassium persulfate or the like, but are not limited thereto. In a prefered embodiment, the thermal polymerization initiator is ammonium persulfate.

Alternatively, when the process is carried out by photochemical polymerisation inside the mould, the polymerization initiator is activated by using radiation source.

The polymerisation process of the invention is carried out in the presence of a polymerization catalyst The term "polymerization catalyst" refers to a catalyst system useful in the polymerization of vinyl monomers to polyvinyl compounds. Examples of polymerization catalyst includes tetramethylethylenediamine.

In an embodiment, optionally in combination with one or more features of the various embodiments described above or below, the cross-linked mould shaped scented product of the invention is that wherein the cross-linked mould shaped polymer (a) is obtainable by the process as defined above, wherein the polymerization is a thermal polymerization, wherein the initiator is ammonium persulfate, and the polymerization catalyst is tetramethylethylenediamine In an embodiment, optionally in combination with one or more features of the various embodiments described above or below, the cross-linked mould shaped scented product of the invention is that wherein the cross-linked mould shaped polymer (a) is obtainable by the process as defined above, wherein the mould is a shaped tridimentional mould.

In an embodiment, optionally in combination with one or more features of the various embodiments described above or below, the cross-linked mould shaped scented product of the invention is that wherein the cross-linked mould shaped polymer (a) is obtainable by the process as defined above, being the monomer (a1) 2-acrylamido-2-methylpropane sulfonic acid.

In an alternative embodiment, optionally in combination with one or more features of the various embodiments described above or below, the cross-linked mould shaped scented product of the invention is that wherein the cross-linked mould shaped polymer (a) is obtainable by the process as defined above, being the monomer (a1) 1-vinyl-2-pyrrolidone.

In an embodiment, optionally in combination with one or more features of the various embodiments described above or below, the cross-linked mould shaped scented product of the invention is that wherein the cross-linked mould shaped polymer (a) is obtainable by the process as defined above, wherein the amount of the monomer (a1) is 100 mol% of the total amount of mols of the monomer (a1), and the monomer (a2).

In a preferred embodiment, optionally in combination with one or more features of the various embodiments described above or below, the cross-linked mould shaped scented product of the invention is that wherein the cross-linked mould shaped polymer (a) is obtainable by the process as defined above, being the monomer (a1) 2-acrylamido-2-methylpropane sulfonic acid and being in an amount of 100 mol% of the total amount of mols of the monomer (a1), and the monomer (a2).

In an alternative embodiment, optionally in combination with one or more features of the various embodiments described above or below, the cross-linked mould shaped scented product of the invention is that wherein the cross-linked mould shaped polymer (a) is obtainable by the process as defined above, being the monomer (a1) 1-vinyl-2-pyrrolidone and being in an amount of 100 mol% of the total amount of mols of the monomer (a1), and the monomer (a2).

In an embodiment, optionally in combination with one or more features of the various embodiments described above or below, the cross-linked mould shaped scented product of the invention is that wherein the cross-linked mould shaped polymer (a) is obtainable by the process as defined above, wherein the amount of the monomer (a1) is comprised from 60 to 80 mol% of the total amount of mols of the monomer (a1), and the monomer (a2); preferably the amount of monomer (a1) is 75 mol% of the total amount of mols of the monomer (a1), and the monomer (a2).

In an alternative embodiment, optionally in combination with one or more features of the various embodiments described above or below, the cross-linked mould shaped scented product of the invention is that wherein the cross-linked mould shaped polymer (a) is obtainable by the process as defined above, being the cross-linker of formula (II) CH₂=CH-CO-NH-CH₂-NH-CO-CH=CH₂.

In an alternative embodiment, optionally in combination with one or more features of the various embodiments described above or below, the cross-linked mould shaped scented product of the invention is that wherein the cross-linked mould shaped polymer (a) is obtainable by the process as defined above, wherein the amount of the cross-linker of formula (II) is comprised from 2 to 5 mol% of the total amount of mols of the monomer (a1), and the monomer (a2); preferably the amount is 2.5 mol%.

In an embodiment, optionally in combination with one or more features of the various embodiments described above or below, the cross-linked mould shaped scented product of the invention is that wherein the amount of the fragrance (b) absorbed in the cross-linked mould shaped polymer (a) is comprised from 1% to 99% by weight of the total weight of the cross-linked mould shaped scented product; preferably comprised from 50% to 95% by weight of the total weight of the product; more preferably comprised from 70% to 90%.

The fragrance of the present invention includes one or more alcohols in combination with one or more essential oils. In an embodiment of the invention, optionally in combination with one or more features of the various embodiments described above or below, the alcohol is a C₂-C₄ alcohol with the proviso that when the article is a perfume, then the alcohol is ethanol. The term "alcohol" refers to a hydrocarbon derivative in which one or more hydrogen atoms have been replaced by one or more -OH group. The term alcohol also includes glycol compounds. In an embodiment of the invention, the alcohol is ethanol, 2-propanol (i.e., isopropanol), or n-propanol; preferably ethanol.

In an embodiment of the invention, optionally in combination with one or more features of the various embodiments described above or below, the fragrance of the present invention includes one or more alcohols in combination with one or more essential oils and further comprises water; thereby a hydroalcoholic mixture has been obtained.

In a particular embodiment, optionally in combination with one or more features of the various embodiments described above or below, the cross-linked mould shaped scented product of the invention is that wherein the fragrance (b) comprises: water in an amount from 10% to 30% by weight of the total weight of the fragrance; C₂-C₄ alcohol in an amount from 70% to 90% by weight of the total weight of the fragrance; and one or more scents in an amount from 1 to 20% by weight of the total weight of the fragrance; being the sum of the weights of the components 100%. In a preferably embodiment, the weight ratio between C₂-C₄ alcohol to water is comprised from 50:50 to 99:1; preferably comprised from 70:30 to 90:10.

In an embodiment of the invention, the cross-linked mould shaped polymer (a) and/or the fragrance (b) of the cross-linked mould shaped scented product of the present invention further comprises one or more acceptable excipients or carriers. Particularly, the acceptable excipients or carriers are selected from the group consisting of colorants, antioxidants, UV (ultra-violet) filters, opacifying agents, solvents, suspending agents, thickeners, and particles.

The cross-linked mould shaped polymer (a) and/or the fragrance (b) of the cross-linked mould shaped scented product of the present invention can include one or more colorants including liposoluble and hydrosoluble colorants. Examples of colorants include, but are not limited to, caramel, Yellow 5, Acid Blue 9/Blue 1, Green 5, Green 3/Fast Green FCF 3, Orange 4, Red 4/Food Red 1, Yellow 6, Acid Red 33/Food Red 12, Red 40, cochineal carmine (CI 15850, CI 75470), Ext. Violet 2, Red 6-7, Ferric Ferrocyanide, Ultramarines, Acid Yellow 3/Yellow 10, Acid Blue 3 or Yellow 10.

The cross-linked mould shaped polymer (a) and/or the fragrance (b) of the cross-linked mould shaped scented product of the present invention mentioned above can include antioxidants. Examples of antioxidants suitable for the present invention include, but are not limited to, butylated hydroxytoluene (BHT), tetrabutyl ethylidinebisphenol, tetrasodium glutamate diacetate, diethylhexyl syringylidene malonate, pentaerythrityl tetra-di-butyl hydroxyhydrocinnamate, tetrasodium glutamate diacetate, tocopherols such as vitamin E and its derivatives such as tocopheryl acetate. Preferably, the antioxidant is BHT.

The cross-linked mould shaped polymer (a) and/or the fragrance (b) of the cross-linked mould shaped scented product of the present invention mentioned above can include UV filters. This term refers to a compound, which chemically or physically absorbs or blocks any type of ultra-violet and/or near visible radiation coming from the sun and from other natural or artificial sources. Examples of UV filters suitable for the present invention, but are not limited to, octocrylene, ethylhexyl triazone, diethylhexyl butamido triazone, ethylhexyl methoxycinnamate, butyl methoxydibenzoylmethane, ethylhexyl salicylate, diethylamino hydroxybenxoyl hexyl benzoate, sodium benzotriazolyl butylphenol sulfonate, and benzotriazolyl dodecyl p-cresol.

The cross-linked mould shaped polymer (a) and/or the fragrance (b) of the cross-linked mould shaped scented product of the present invention can include one or more opacifying. The term "opacifying agent" refers to a compound that becomes a product opaque or that enhances the opacity of said product. Examples of opacifying agent suitable for the present invention, but are not limited to, titanium dioxide, ozokerite, cetearyl Alcohol, mica, tin oxide, cera microcristallina, ceresin, silica, cetyl alcohol, talc, styrene/acrylates copolymer, stearyl alcohol, hydrated silica, glycol distearate, myristyl myristate, nylon-12, glycol stearate, boron nitride, kaolin, magnesium aluminum silicate, alumina, propylene glycol stearate SE, methyl methacrylate, and mixture thereof.

The cross-linked mould shaped polymer (a) and/or the fragrance (b) of the cross-linked mould shaped scented product of the present invention can include one or more solvents including dipropylene glycol, propylene glycol, isopropyl myristate, ethylhexyl, palmitate, diethyl phthalate, triethyl citrate, isododecane, isohexadecane, isoeicosane, isoparaffin fluids, C₁₃-C₃₀ alkanes, hydrogenated godecene, hydrogenated gododecene, hydrogenated polydecene, hydrogenated polydodecene, hydrogenated tridodecene, hydrogenated polyisobutene, mineral oils, and mixture thereof. The solvent can also be a linear silicone such as for example dimethicones, cyclic silicones, and mixture thereof.

The cross-linked mould shaped polymer (a) and/or the fragrance (b) of the cross-linked mould shaped scented product of the present invention can include one or more suspending agents. The term "suspending agent" refers to a compound capable of stabilizing suspended particles in an alcoholic or hydroalcoholic medium. Examples of suspending agents suitable of the present invention include CARBOPOL 980 and Aristoflex TAC.

The cross-linked mould shaped polymer (a) and/or the fragrance (b) of the cross-linked mould shaped scented product of the present invention can include one or more thickeners. The term "thickener" refers to an agent that enhance the viscosity of the compound to which it is added. Examples of thickeners suitable for the present invention includes, but are not limited to fumed silica, colloidal silica, calcium silicate, gelatinized starch, microcrystalline cellulose, talc, magnesium stearate, polyacrylate cross-polymer-6, Ultrathix P-100, manufactured by ISP. Ultrathix P-10The Aristoflex TAC, Carbopol 980, Sepimax Zen, Ultrathix P-100 and mixture thereof.

The cross-linked mould shaped polymer (a) and/or the fragrance (b) of the cross-linked mould shaped scented product of the present invention can include one or more particles. The term "particle" refers to a discrete portion of material that has mass and dimension being elements of a suspension. The term particle includes from nanoparticles to macroparticles. Particularly, nanoparticles refers to those particles having a diameter comprised from 1 to 1000 nm. The term microparticles are also included in the term particles and refers to those having a diameter comprised from 1 to 1000 microns. And, macroparticles are those having a diameter comprised from 1001 microns to 1cm.

In an embodiment of the invention, the particles can modify the visual appearance of the scented article. Examples of these particles includes, but not limited to, sparkling particles such as glitter; reflective particles; encapsulated particles; microsphere enclosing color, or opacity, or light emitting such as tiny light, emitting diodes, LED's, or luminescent materials, or reflecting particles.

The cross-linked mould shaped polymer (a) and/or the fragrance (b) of the cross-linked mould shaped scented product of the present invention may contain other ingredients appropriate for the treatment of the skin to which the product of the invention is applied. Particularly, other ingredients suitable for the present invention include, but not limited to, moisturizing agents, and skin barrier recovery agent.

The term "hydrating agent" or "moisturizer" or "moisturizing agent" which is herein used interchangeably refers to a material which increases the water content of the skin and helps keep it soft and smooth. Examples of appropriate moisturizing agents include, but are not limited to, aloe vera, althea, avocado oil, balm mint, burdock, shea butter, camphor, chamomile, and isodecyl oleate, collagen, collagen amino acids, dimethiconol, glycine, hyaluronic acid, dimethylsilanol hyaluronate, magnesium stearate, maltitol, maltose, pyrrolidone carboxylic acid (PCA), manganese PCA, sodium PCA, mannitol, trehalose, trilactin, glucose, glutamic acid, hydrolyzed caesalpinia spinosa gum, caesalpinia spinosa gum, prunus persica extract, prunus serotina extract, echinacea angustifolia extract, Echinacea purpurea extract, methyl gluceth, hydrolyzed wheat gluten, erythritol, aluminium stearoyl glutamate, copper acetylmethionate, and ditridecyl dimmer dilinoleate.

The term "skin barrier recovery agent" refers to material whose composition and/or structure are similar to the skin barrier allowing the reparation of its deficiencies. This term includes anti-wrinkle agents, and de-pigmentation agents. Examples of appropriate topical regenerating skin agents include, but are not limited to, ceramides, cholesterol, fatty acids, and precursors of these lipids including cerebrosides, sphingoid bases such as phytosphingosine or sphingosine, or phospholipids including phosphatidylcholine, and agents that promote the synthesis of epidermal lipids like urea, dexpanthenol, and alpha-hydroxyacids including lactic acid among others.

In an embodiment of the invention, the cross-linked mould shaped polymer (a) further comprises one or more acceptable excipients or carriers. Those excipients or carriers can be introduced inside the cross-linked mould shaped polymer (a) during the polymerizing process carried out in the mould; or alternatively by putting in contact the cross-linked mould shaped polymer (a) in a fragrance which comprises the above mentioned excipients or carriers

Other aspect of the invention relates to a process for the preparation of the cross-linked shaped scented product of the present invention. The process for preparing the cross-linked mould shaped scented product as defined above or below comprises: (a) Disolving in water a monomer (a1), a monomer (a2), and a cross-linker; (b) Adding a polymerization initiator and a polymerization catalyst to the dissolution obtained in step (a); (c) Moulding the mixture obtained in step (b) in a shaped mould; (d) Polymerizing the moulded mixture obtained in step (c) to obtain the cross-linked mould shaped polymer; (e) Removing the cross-linked mould shaped polymer obtained in step (d); (f) Cleaning the cross-linked mould shaped polymer obtained in step (e); (g) Drying the cross-linked mould shaped polymeric compound obtained in step (f); and (h) Putting in contact the cross-linked mould shaped polymeric compound obtained in step (g) with an alcoholic fragrance as defined above or below.

All the embodiments disclosed above for the vinyl compounds, the cross-linking agent of formula (II), the polymerization inititiator, the polymerization catalyst, as well as for the experimental conditions of the polymerization step defined above for the first aspect of the invention applies also for the process for the preparation of the cross-linked mould shaped scented product of the second aspect of the invention.

The third aspect of the invention relates to a scented article, which comprises the product as defined above. The cross-linked mould shaped scented product of the invention can be advantageously used in all the fields or modern perfumery to positively impart or modify the smell of a product into which they are applied.

In an embodiment, the scented article of the invention is in form of an air freshener. The term "air freshener" refers to a scented article, which comprises the cross-linked mould shaped scented product of the invention including the fragrance (b) as defined above, which is appropriate for putting in contact with the air of a space, being the space a closed internal space such as for example rooms and cupboards.

Air fresheners introduce fragrance into the air of the spaces either as droplets which transition to vapor, or as the molecules of fragrance ingredients directly evaporating from a source. Then, fragrances diffuse into the air to mask other odors or to introduce a specific odor.

In an embodiment, the scented article of the invention is in form of a jewel. The term "jewel" refers to a scented article, which comprises the cross-linked mould shaped scented product of the invention including the fragrance (b) as defined above, which is appropriate for being worn, for example earings, a bracelet or a ring. Then, fragrances diffuse from the jewel into the air to perfum the skin and the surrounding air.

In an embodiment, the scented article of the invention comprises the cross-linked mould shaped scented product as defined above and further comprises a perfume. In a particular embodiment, the perfume is selected from the group consisting of eau de perfume, eau de cologne, and eau de toilette. These scented articles can be applied to a surface, being the surface an inert, or body surface. Examples of inert surface can be selected from textile surfaces, plastic surfaces or natural origin surfaces such as wood or paper.

The term "body surface" refers to any surface of the human or animal body, which may serve as a substrate for applying the composition of the invention. Examples of body surface include human skin or hair, and animal skin or fur.

In an embodiment of the invention, the scented articles of the present invention further comprises one or more acceptable excipients or carriers. Particularly, the acceptable excipients or carriers are selected from the group consisting of colorants, antioxidants, UV filters, opacifying agents, solvents, suspending agents, thickeners, and particles. All the embodiments disclosed above for the acceptable excipients or carriers applies also for the scented articles of the second aspect of the invention.

The scented articles of the present invention can be prepared according to methods well known in the state of the art. The appropriate excipients and/or carriers, and their amounts, can readily be determined by those skilled in the art according to the type of scented article being prepared.

Other aspect of the invention relates to a process for the preparation of the scented article defined above. In an embodiment, wherein when the scented article is in form of an air freshener or a jewel, then the process comprises carrying out the process as defined above comprising steps (a), (b), (c), (d), (e), (f), (g) and (h). In an embodiment, the process further comprises finishing conditioning steps such as for example brighten steps, and design steps.

In an alternative embodiment, wherein when the scented article of the invention further comprises a perfume, then the process comprises carrying out the process as defined above comprising steps (a), (b), (c), (d), (e), (f), (g) and (h), wherein the step (h) comprises introducing the cross-linked mould shaped polymer (a) obtained in step (g) in a recipient, for instance a bottle. In a preferred embodiment, step (h) comprises introducing the cross-linked mould shaped polymer (a) in the recipient and filling the recipient with the fragrance; or alternatively, filling the recipient with the fragrance, and introducing the cross-linked mould shaped polymer (a). In an embodiment, the process further comprises finishing steps such as for example introducing one or more additional acceptable excipients or carriers as defined above.

Other aspect of the invention relates to the use of the cross-linked shaped scented product of the first aspect of the invention; or alternatively the scented article as defined in the third aspect of the invention for perfuming.

Throughout the description and claims the word "comprise" and variations of the word, are not intended to exclude other technical features, additives, components, or steps. Furthermore, the word "comprise" encompasses the case of "consisting of". Additional objects, advantages, and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### 1. CROSS-LINKED POLYMERS

### 1A. Composition of the cross-linked polymers

The qualitative and quantitative composition of the cross-linked mould shaped polymers examples 1-3 of the present invention and the comparative cross-linked polymer example 4 is shown below.

### Example 1: Cross-linked mould shaped polymer of AMPS:

Monomer (a1): 2-acrylamido-2-methylpropane sulfonic acid
Amount of monomer (a1): 100 mol %.
Cross-linker of formula (II): CH₂=CH-CO-NH-CH₂-NH-CO-CH=CH₂
Amount of cross-linker: 2.5 mol% of the total mols of monomer (a1)

### Example 2: Cross-linked mould shaped polymer of NVP:

Monomer (a1): 1-vinyl-2-pyrrolidone
Amount of monomer (a1): 100 mol %.
Cross-linker of formula (II): CH₂=CH-CO-NH-CH₂-NH-CO-CH=CH₂
Amount of cross-linker: 2.5 mol% of the total mols of monomer (a1)

### Example 3: Cross-linked mould shaped polymer of AMPS and acrylic acid:

Monomer (a1): 2-acrylamido-2-methylpropane sulfonic acid
Amount of monomer (a1): 75 mol %.
Monomer (a2): acrylic acid
Amount of monomer (a2): 25 mol %.
Cross-linker of formula (II): CH₂=CH-CO-NH-CH₂-NH-CO-CH=CH₂
Amount of cross-linker: 2.5 mol% of the total amount of mols ((a1) and (a2)).

### Comparative Example 4: cross-linked polymer of AMPS:

Monomer (a1): 2-acrylamido-2-methylpropane sulfonic acid
Amount of monomer (a1): 100 mol %.
Cross-linker of formula (II): CH₂=CH-CO-NH-CH₂-NH-CO-CH=CH₂
Amount of cross-linker: 5 mol% of the total mols of monomer (a1)

### 1B. Preparation Process of the cross-linked polymer

### 1B.1. Cross-linked mould shaped polymer of the invention

### Polymers of 2-acrylamido-2-methylpropane sulfonic acid

The cross-linked mould shaped polymer of Example 1 disclosed in section 1A was prepared following the process disclosed below.
STEP A:
   a(i) To a mixture of 5g de of the total mols of monomer (AMPS) and 0.092g of N,N'-methylenebis(acrylamide), 25ml of deionized water was added; and the resulting mixture was stirred;
   a(ii) The mixture obtained in step a(i) was degassed by argon gas bubbling through it for 10-15 minutes;
STEP B: To the mixture obtained in step a(ii), 0.04g of ammonium persulfate was added slowly; and to the resulting mixture 0.06g of tetramethylethylenediamine was added;
STEP C: The mixture obtained in step B is moulded in a cubic mould;
STEP D: The polymerization of the moulded mixture of step C was carried out at 70°C for 20h to obtain a cross-linked moulded polymer;
STEP E: The cross-linked mould shaped polymer of step D was removed from the mould;
STEP F: the cross-linked mould shaped polymer of step E was washed by immersing the polymer in water for at least 24h;
STEP G: the cross-linked mould shaped polymer of step F was dried at 50°C until constant weight.

The cross-linked mould shaped polymer obtained in Step G is a solid.

### Polymers of 1-vinyl-2-pyrrolidone

The cross-linked mould shaped polymer of Example 2 disclosed in section 1A was prepared following the process disclosed below.
STEP A:
   a(i) To a mixture of 2.5g of 1-vinyl-2-pyrrolidone and 0.089g of N,N'-methylenebis(acrylamide), 10ml of deionized water was added; and the resulting mixture was stirred;
   a(ii) The mixture obtained in step a(i) was degassed by argon gas bubbling through it for 10-15 minutes;
STEP B: To the mixture obtained in step a(ii), 0.02g of ammonium persulfate was added slowly; and to the resulting mixture 0.03g of tetramethylethylenediamine was added;
STEP C: The mixture obtained in step B is moulded in a cubic mould;
STEP D: The polymerization of the moulded mixture of step C was carried out at 70°C for 20h to obtain a cross-linked moulded polymer;
STEP E: The cross-linked mould shaped polymer of step D was removed from the mould;
STEP F: the cross-linked mould shaped polymer of step E was washed by immersing the polymer in water for 24h;
STEP G: the cross-linked mould shaped polymer of step F was dried at 50°C until constant weight.

The cross-linked mould shaped polymer obtained in Step G is a solid.

### Polymers of 2-acrylamido-2-methylpropane sulfonic acid and acrylic acid

The cross-linked mould shaped polymer of Example 3 disclosed in section 1A was prepared following the process disclosed below.
STEP A:
   a(i) To a mixture of 3.73g of 2-acrylamido-2-methylpropane sulfonic acid, 0.43 of acrylic acid, and 0.092g of N,N'-methylenebis(acrylamide), 25ml of deionized water was added; and the resulting mixture was stirred;
   a(ii) The mixture obtained in step a(i) was degassed by argon gas bubbling through it for 10-15 minutes;
STEP B: To the mixture obtained in step a(ii), 0.04g of ammonium persulfate was added slowly; and to the resulting mixture 0.06g of tetramethylethylenediamine was added;
STEP C: The mixture obtained in step B is moulded in a cubic mould;
STEP D: The polymerization of the moulded mixture of step C was carried out at 70°C for 20h to obtain a cross-linked moulded polymer;
STEP E: The cross-linked mould shaped polymer of step D was removed from the mould;
STEP F: the cross-linked mould shaped polymer of step E was washed by immersing the polymer in water for 24h;
STEP G: the cross-linked mould shaped polymer of step F was dried at 50°C until constant weight.

The cross-linked mould shaped polymer obtained in Step G is a solid.

### 1B.2. Comparative cross-linked polymer

The cross-linked polymer of Comparative Example 4 disclosed in section 1A was prepared following the process disclosed below.

### 1.B.2.1 cross-linked Shaped comparative polymer.

STEP A:
   a(i) To a mixture of 10g of 2-acrylamido-2-methylpropane sulfonic acid, and 0.35g of N,N'-methylenebis(acrylamide), 50ml of deionized water was added; and the resulting mixture was stirred;
   a(ii) The mixture obtained in step a(i) was degassed by argon gas bubbling through it for 10-15 minutes;
STEP B: To the mixture obtained in step a(ii), 0.11g of ammonium persulfate was added slowly; and to the resulting mixture 0.18g of tetramethylethylenediamine was added;
STEP C: The mixture obtained in step B is moulded in identical cubic moulds;
STEP D: The polymerization of the moulded mixture of step C was carried out at 70°C for 20h to obtain a cross-linked cubic polymer;
STEP E: The cross-linked shaped cubic polymers of step D were removed from the moulds;
STEP F: cross-linked cubic polymers of step E were washed by immersing the polymers into water;
STEP G: cross-linked cubic polymers of step F were dried until constant weight of 0.5-0.6g.

### 1.B.2.2 Powdered comparative cross-linked polymer.

STEP H: Some of the cubes obtained in step G were crushed with a basic analytical grinding mill to obtain a powder. The powder thus obtained is sieved by a sieve of diameter less than 150 µm.

### 2. PRODUCTS

### 2A. Composition of the tested products

The qualitative and quantitative composition of a cross-linked mould shaped scented product containing a cross-linked polymer defined in section 1A and an alcoholic fragrance inside the cross-linked polymer is shown below.

The amount of the fragrance inside the cross-linked polymer is expressed in grams of fragrance absorbed per gram of cross-linked polymer.

The composition of the fragrance is as follows:
- 85 % by weight of the weight of the fragrance of a mixture of ethanol and water, wherein the weight ratio between ethanol and water is 80:20; and
- 15 % by weight of the weight of the fragrance of one or more ingredients.

### Comparative cross-linked shaped scented product 1

Product 1 comprises the cross-linked shaped polymer of Example 1, obtained by 1B.2.1 preparation process, having 85 grams of fragrance absorbed per gram of polymer.

### Comparative cross-linked scented product 2 in gel form.

Comparative Product 2 comprises powdered cross-linked polymer of Comparative Example 4, obtained by 1B.2.2 preparation process, having 85 grams of fragrance absorbed per gram of polymer.

### 2B. Preparation Process

### Cross-linked mould shaped scented Product 1

Product 1 was prepared by immersing the cross-linked cubic-shaped polymer of example 1 of invention disclosed in section 1A, following the process of section 1B.1 step G, in the fragrance as defined in section 2A for 48 hours.

The resulting cross-linked mould shaped scented product 1 is a solid.

### Comparative cross-linked scented Product 2 in gel form

Product 2 was prepared by immersing the powdered cross-linked polymer of example 1 of invention disclosed in section 1A, following the process of section 1B.2 step H, in the fragrance as defined in section 2A for 48 hours.

The resulting comparative cross-linked scented product 2 is a gel (that is a semi-solid form).

### 3. EVAPORATION TEST

### 3.1. Material

0.54 g of cross-linked cubic-shaped scented Product 1 obtained by process 1.B.1

0.54 g of Comparative cross-linked scented product 2 in powder form obtained by process 1.B.2

### 3.2. Method

Tested Products were deposited in a Petri plate of 14cm of diameter. Plates having tested products were opened and were left in a place having a constant temperature, humidity, and airflows for an appropriate time to absorb about 40 g of fragrance each product.

The tested Petri plates were weight at time zero, and at each evaporating tested time up to 166 hours.

### 3.3. Results

Table 1 shows the evaporation profile of the fragrance from the tested product. The weight loss of the product is expressed in %

**Table 1**

| Time (h) | % of weight loss | |
|---|---|---|
| | cross-linked shaped Scented Product 1 | Comparative Product 2 |
| 0 | 0 | 0 |
| 20 | 42 | 62 |
| 30 | 60 | 85 |
| 50 | 75 | 87 |
| 100 | 85 | 90 ⁽¹⁾ |
| 166 | 87 | 90 ⁽¹⁾ |

| | | |
|---|---|---|
| (1) 10% of the amount corresponds to non-volatile compounds of the fragrance included inside the products of the invention. The non-volatile compounds do not release from the product and they rest inside the product | | |

Results of Table 1 show that only the product of the invention, which comprises the cross-linked mould shaped polymer (a) and a fragrance (b) absorbed inside allows prolonging the release of the volatile ingredients of the fragrance.

As it is showed in the results of the Table 1, less than 50% of the volatile ingredients of the fragrance are released from the product of the invention after 20 hours of the beginning of the exposure and at least 25% of the volatile ingredients of the fragrance are retained inside the product of the invention after 50 hours. In comparison with the comparative compositions of the state of the art, the results of the Table 1 also show that, the product of the invention has already retained volatile ingredients inside the product of the invention after 166 hours (that is 3% by weight of volatile compounds inside of the product of the invention).

It is advantageous because it allows having a continuous release of the fragrance prolonging a pleasant odour organoleptic effect even after 100 hours of exposure.

### REFERENCES CITED IN THE APPLICATION

1. Varma, A. J., et al. "Separations based on chemically selective polymer gels". Chemical Engineering Science, 1995, vol. 50 (23), pp 3835-3838.
2. Graham, N. B., et al,. "Interaction of poly(ethylene oxide) with solvents; 1. Preparation and swelling of a crosslinked poly(ethylene oxide) hydrogel".Polymer, 1982, vol. 23, pp. 1345.
3. David J. Rowe. "Chemistry and technology of flavors and fragrances". Chapter 1. "Introduction", Blackwell Publishing, 2005, pp.1-11

## Claims

1. A cross-linked mould shaped scented product comprising:
(a) a cross-linked mould shaped polymer; and
(b) an alcoholic fragrance absorbed inside the polymer;
wherein:
the cross-linked mould shaped scented product is capable of modifying its volume, and maintaining its shape when the alcoholic fragrance is released; and
the cross-linked mould shaped polymer (a) is capable of modifying its volume, and maintaining its shape when the alcoholic fragrance is absorbed and is obtainable by a process comprising polymerizing in a shaped mould:
(a1) a monomer containing a vinyl group and an amide group selected from the group consisting of 2-acrylamido-2-methylpropane sulfonic acid and 1-vinyl-2-pyrrolidone; and
(a2) a monomer of formula (I):
CH₂=CH-R₁ (I)
in the presence of a crosslinker of formula (II):
CH₂=CH-R₂-CH=CH₂ (II)
a polymerization initiator; and a polymerization catalyst;
wherein:
R₁ is selected from the group consisting of -CN, -COR₃, 2-pyrrolidone and-CO-NH-C(CH₃)₂-CH₂-SO₃H;
R2 is selected from the group consisting of -CO-NH-CH₂-NH-CO- and -CO-NH-[CH₂-O-]ₙNH-CO-;
R₃ is OR₄, or NHR₅;
R₄ is selected from the group consisting of H, -CH₂-CH₂-OH, and -CH₂-CH(OH)-CH₂-OH;
R₅ is selected from the group consisting of H, and -C(CH₃)₂-CH₂-SO₃H;
n is an integer selected from 1, 2, and 3;
the amount of the monomer (a1) is comprised from 50 to 100 mol% of the total amount of mols of the monomer (a1) and the monomer (a2);
the amount of the monomer (a2) is comprised from 0 to 50 mol% of the total amount of mols of the monomer (a1) and the monomer (a2);
the amount of crosslinker is comprised from 1 to 10 mol % of the sum of the amount of mols of the monomer (a1) and the monomer (a2), and
being the sum of the amounts of the monomer (a1) and the monomer (a2) equal to 100 mol%;
provided that:
when the monomer (a1) is 2-acrylamido-2-methylpropane sulfonic acid, then the monomer (a2) is other than 2-acrylamido-2-methylpropane sulfonic acid and
when the monomer (a1) is1-vinyl-2-pyrrolidone, then the monomer (a2) is other than 1-vinyl-2-pyrrolidone.

2. The product according to claim 1, wherein the amount of crosslinker is comprised from 2 to 5 mol % of the sum of the amount amount of mols of the monomer (a1), and the monomer (a2).

3. The product according to any of the claims 1-2, wherein the amount of the monomer (a1) is 100 mol% of the total amount of mols of the monomer (a1), and the monomer (a2).

4. The product according to any of the claims 1-2, wherein the amount of the monomer (a1) is comprised from 60 to 80 mol% of the total amount of mols of the monomer (a1) and the monomer (a2).

5. The product according to any of the claims 1-4, wherein the compound of formula II is CH₂=CH-CO-NH-CH₂-NH-CO-CH=CH₂.

6. The product according to any of the claims 1-5, wherein the amount of the fragrance (b) absorbed in the cross-linked mould shaped polymer (a) is comprised from 1% to 99% by weight of the total weight of the product.

7. The product according to claim 6, wherein the amount of the fragrance (b) absorbed in the cross-linked mould shaped polymer (a) is comprised from 80% to 99% by weight of the total weight of the product.

8. The product according to any of the claims 1-7, wherein the cross-linked mould shaped polymer (a) and/or the fragrance (b) further comprises one or more acceptable excipients or carriers.

9. A scented article comprising the product as defined in any of the claims 1-8.

10. The product according to claim 8, or the scented article according claim 9, wherein the one or more appropriate acceptable excipients or carriers are selected from the group consisting of colorants, antioxidants, ultra-violet filters, opacifying agents, solvents, suspending agent, thickener, and particles.

11. A process for preparing the product as defined in any of the claims 1-8 comprising:
(a) Dissolving in water a monomer (a1), a monomer (a2), and a cross-linker;
(b) Adding a polymerization initiator and a polymerization catalyst to the dissolution obtained in step (a);
(c) Moulding the mixture obtained in step (b) in a shaped mould;
(d) Polymerizing the moulded mixture obtained in step (c) to obtain the cross-linked mould shaped polymer;
(e) Removing the cross-linked mould shaped polymer obtained in step (d);
(f) Cleaning the cross-linked mould shaped polymer obtained in step (e);
(g) Drying the cross-linked mould shaped polymer obtained in step (f); and
(h) Putting in contact the cross-linked mould shaped polymer obtained in step (g) with an alcoholic fragrance.

12. Use of the product as defined in any of the claims 1-8 or 10; or alternatively the scented article as defined in any of the claims 9-10 for perfuming.

## Patentansprüche

1. Ein vernetztes, in einer Form geformtes, duftendes Produkt umfassend:
(a) ein vernetztes, in einer Form geformtes Polymer; und
(b) einen innerhalb des Polymer absorbierten alkoholhaltigen Duft;
wobei:
das vernetzte, in einer Form geformte, duftende Produkt sein Volumen modifizieren kann, und dabei seine Form behält, wenn der alkoholhaltige Duft freigesetzt wird; und
das vernetzte, in einer Form geformte Polymer (a) sein Volumen modifizieren kann, und dabei seine Form behält, wenn der alkoholhaltige Duft absorbiert wird und mittels eines Verfahrens erhalten werden kann, das die Polymerisation umfasst von:
(a1) einem Monomer, das eine Vinylgruppe und eine Amidgruppe enthält und ausgewählt ist aus der Gruppe bestehend aus 2-Acrylamido-2-methylpropansulfonsäure und 1-Vinyl-2-pyrrolidon; und
(a2) einem Monomer der Formel (I):
CH₂=CH-R₁ (I)
in Gegenwart eines Vernetzers der Formel (II):
CH₂=CH-R₂-CH=CH₂ (II)
eines Initiators der Polymerisation; und eines Katalysators der Polymerisation;
wobei:
R₁ ausgewählt aus der Gruppe bestehend aus -CN, -COR₃, 2-Pyrrolidon und -CO-NH-C(CH₃)₂-CH₂-SO₃H ist;
R₂ ausgewählt aus der Gruppe bestehend aus -CO-NH-CH₂-NH-CO- und-CO-NH-[CH₂-O-]ₙNH-CO- ist;
R₃ = OR₄ oder NHR₅ ist;
R₄ ausgewählt aus der Gruppe bestehend aus H, -CH₂-CH₂-OH, und -CH₂-CH(OH)-CH₂-OH ist;
R₅ ausgewählt aus der Gruppe bestehend aus H, und -C(CH₃)₂-CH₂-SO₃H ist;
n eine Ganzzahl ausgewählt aus 1, 2 und 3 ist;
die Menge des Monomers (a1) von 50 Mol-% bis 100 Mol.-% bezogen auf die gesamte Molenmenge des Monomers (a1) und des Monomers (a2) reicht;
die Menge des Monomers (a2) von 0 Mol-% bis 50 Mol.-% bezogen auf die gesamte Molenmenge des Monomers (a1) und des Monomers (a2) reicht;
die Menge des Vernetzers von 1 Mol-% bis 10 Mol.-% bezogen auf die Summe der Molenmenge des Monomers (a1) und des Monomers (a2) reicht, und
die Summe der Mengen des Monomers (a1) und des Monomers (a2) 100 Mol.-% gleicht;
unter der Voraussetzung, dass:
wenn das Monomer (a1) 2-Acrylamido-2-methylpropansulfonsäure ist, das Monomer (a2) verschieden von 2-Acrylamido-2-methylpropansulfonsäure ist und
wenn das Monomer (a1) 1-Vinyl-2-pyrrolidon ist, das Monomer (a2) verschieden von 1-Vinyl-2-pyrrolidon ist.

2. Das Produkt nach Anspruch 1, wobei die Menge des Vernetzers von 2 Mol-% bis 5 Mol.-% bezogen auf die Summe der Molenmenge des Monomers (a1) und des Monomers (a2) reicht.

3. Das Produkt nach einem der Ansprüche 1-2, wobei die Menge des Monomers (a1) 100 Mol.-% bezogen auf die gesamte Molenmenge des Monomers (a1) und des Monomers (a2) ist.

4. Das Produkt nach einem der Ansprüche 1-2, wobei die Menge des Monomers (a1) von 60 Mol-% bis 80 Mol.-% bezogen auf die gesamte Molenmenge des Monomers (a1) und des Monomers (a2) reicht.

5. Das Produkt nach einem der Ansprüche 1-4, wobei die Verbindung der Formel II CH₂=CH-CO-NH-CH₂-NH-CO-CH=CH₂ ist.

6. Das Produkt nach einem der Ansprüche 1-5, wobei die Menge des im vernetzten, in einer Form geformten Polymer (a) absorbierten Duftes (b) von 1 Gew.-% bis 99 Gew.-% bezogen auf das gesamte Gewicht des Produkts reicht.

7. Das Produkt nach Anspruch 6, wobei die Menge des im vernetzten, in einer Form geformten Polymer (a) absorbierten Duftes (b) von 80 Gew.-% bis 99 Gew.-% bezogen auf das gesamte Gewicht des Produkts reicht.

8. Das Produkt nach einem der Ansprüche 1-7, wobei das vernetzte, in einer Form geformte Polymer (a) und/oder der Duft (b) weiterhin eine(n) oder mehr akzeptable Hilfsstoffe oder Trägersubstanzen umfasst.

9. Ein Duftartikel umfassend das Produkt wie in einem der Ansprüche 1-8 definiert.

10. Das Produkt nach Anspruch 8, oder das Duftartikel nach Anspruch 9, wobei der beziehungsweise die eine oder die mehreren geeigneten akzeptablen Hilfsstoffe oder Trägersubstanzen ausgewählt sind aus der Gruppe bestehend aus Farbstoffen, Antioxidationsmitteln, UV-Filtern, Trübungsmitteln, Lösungsmitteln, Suspensionsmittel, Verdickungsmittel und Partikeln.

11. Ein Verfahren zur Herstellung des Produkts wie in einem der Ansprüche 1-8 definiert umfassend:
(a) das Lösen von einem Monomer (a1), einem Monomer (a2) und einem Vernetzer in Wasser;
(b) das Hinzugeben von einem Initiator der Polymerisation und einem Katalysator der Polymerisation der in Schritt (a) erhaltenen Lösung;
(c) das Formen der in Schritt (b) erhaltenen Mischung in einer geformten Form;
(d) die Polymerisation der in Schritt (c) erhaltenen Mischung, um das vernetzte, in einer Form geformte Polymer zu erhalten;
(e) das Entfernen des in Schritt (d) erhaltenen vernetzten, in einer Form geformten Polymers;
(f) das Reinigen des in Schritt (e) erhaltenen vernetzten, in einer Form geformten Polymers;
(g) das Trocknen des in Schritt (f) erhaltenen vernetzten, in einer Form geformten Polymers; und
(h) das In-Kontakt-Bringen des in Schritt (g) erhaltenen vernetzten, in einer Form geformten Polymers mit einem alkoholhaltigen Duft.

12. Verwendung des Produkts wie in einem der Ansprüche 1-8 oder 10 definiert; oder, ersatzweise, des Duftartikels wie in einem der Ansprüche 9-10 definiert zur Parfümierung.

## Revendications

1. Un produit parfumé formé dans un moule et réticulé comprenant :
(a) un polymère formé dans un moule et réticulé ; et
(b) un parfum alcoolique absorbé dans le polymère;
dans lequel :
le produit parfumé formé dans un moule et réticulé est capable de modifier son volume, et maintenant sa forme, lorsque le parfum alcoolique est libéré ; et
le polymère formé dans un moule et réticulé (a) est capable de modifier son volume, et maintenant sa forme, lorsque le parfum alcoolique est absorbé et peut être obtenu moyennant un procédé comprenant la polymérisation, dans un moule ayant une forme donnée, de :
(a1) un monomère contenant un groupe vinyle et un groupe amide choisi dans le groupe constitué de l'acide 2-acrylamido-2-méthylpropanesulfonique et la 1-vinyl-2-pyrrolidone ; et
(a2) un monomère de formule (I) :
CH₂=CH-R₁ (I)
en présence d'un agent de réticulation de formule (II) :
CH₂=CH-R₂-CH=CH₂ (II)
un initiateur de la polymérisation ; et un catalyseur de la polymérisation ;
où :
R₁ est choisi dans le groupe constitué de -CN, -COR₃, 2-pyrrolidone et -CO-NH-C(CH₃)₂-CH₂-SO₃H ;
R₂ est choisi dans le groupe constitué de -CO-NH-CH₂-NH-CO- et -CO-NH-[CH₂-O-]ₙNH-CO- ;
R₃ est OR₄, ou NHR₅ ;
R₄ est choisi dans le groupe constitué de H, -CH₂-CH₂-OH, et -CH₂-CH(OH)-CH₂-OH ;
R₅ est choisi dans le groupe constitué de H, et -C(CH₃)₂-CH₂-SO₃H ;
n est un nombre entier choisi de 1, 2 et 3 ;
la quantité du monomère (a1) est comprise de 50 à 100 % molaire de la quantité totale de moles du monomère (a1) et du monomère (a2) ;
la quantité du monomère (a2) est comprise de 0 à 50 % molaire de la quantité totale de moles du monomère (a1) et du monomère (a2) ;
la quantité d'agent de réticulation est comprise de 1 à 10 % molaire de la somme de la quantité de moles du monomère (a1) et du monomère (a2), et
étant la somme des quantités du monomère (a1) et du monomère (a2) égale à 100 % molaire ;
à condition que :
lorsque le monomère (a1) est l'acide 2-acrylamido-2-méthylpropanesulfonique, alors le monomère (a2) ne soit pas l'acide 2-acrylamido-2-méthylpropanesulfonique et
lorsque le monomère (a1) est 1-vinyl-2-pyrrolidone, alors le monomère (a2) ne soit pas la 1-vinyl-2-pyrrolidone.

2. Le produit selon la revendication 1, dans lequel la quantité d'agent de réticulation est comprise de 2 à 5 % molaire de la somme de la quantité de moles du monomère (a1) et du monomère (a2).

3. Le produit selon l'une quelconque des revendications 1-2, dans lequel la quantité du monomère (a1) est 100 % molaire de la quantité totale de moles du monomère (a1) et du monomère (a2).

4. Le produit selon l'une quelconque des revendications 1-2, dans lequel la quantité du monomère (a1) est comprise de 60 à 80 % molaire de la quantité totale de moles du monomère (a1) et du monomère (a2).

5. Le produit selon l'une quelconque des revendications 1-4, dans lequel le composé de formule II est CH₂=CH-CO-NH-CH₂-NH-CO-CH=CH₂.

6. Le produit selon l'une quelconque des revendications 1-5, dans lequel la quantité du parfum (b) absorbé dans le polymère formé dans un moule et réticulé (a) est comprise de 1 % à 99 % en poids par rapport au poids total du produit.

7. Le produit selon la revendication 6, dans lequel la quantité du parfum (b) absorbé dans le polymère formé dans un moule et réticulé (a) est comprise de 80 % à 99 % en poids par rapport au poids total du produit.

8. Le produit selon l'une quelconque des revendications 1-7, dans lequel le polymère formé dans un moule et réticulé (a) et/ou le parfum (b) comprend en outre un ou plusieurs excipients ou véhicules acceptables.

9. Un article parfumé comprenant le produit tel que défini dans l'une quelconque des revendications 1-8.

10. Le produit selon la revendication 8, ou l'article parfumé selon la revendication 9, dans lequel l'un ou les plusieurs excipients ou véhicules acceptables appropriés sont choisis dans le groupe constitué de colorants, antioxydants, filtres ultraviolet, opacifiants, solvants, agent de suspension, épaississant et particules.

11. Un procédé de préparation du produit tel que défini dans l'une quelconque des revendications 1-8 comprenant :
(a) Dissoudre un monomère (a1), un monomère (a2), et un agent de réticulation dans l'eau ;
(b) Ajouter un initiateur de la polymérisation et un catalyseur de la polymérisation à la dissolution obtenue dans l'étape (a) ;
(c) Mouler le mélange obtenu dans l'étape (b) dans un moule ayant une forme donnée ;
(d) Polymériser le mélange moulé obtenu dans l'étape (c) afin d'obtenir le polymère formé dans un moule et réticulé ;
(e) Enlever le polymère formé dans un moule et réticulé obtenu dans l'étape (d) ;
(f) Nettoyer le polymère formé dans un moule et réticulé obtenu dans l'étape (e) ;
(g) Sécher le polymère formé dans un moule et réticulé obtenu dans l'étape (f); et
(h) Mettre en contact le polymère formé dans un moule et réticulé obtenu dans l'étape (g) avec un parfum alcoolique.

12. Utilisation du produit tel que défini dans l'une quelconque des revendications 1-8 ou 10 ; ou, alternativement, de l'article parfumé tel que défini dans l'une quelconque des revendications 9-10 pour parfumer.
